# DEMANDE DE BREVET EUROPEEN

(11) **EP 1 559 404 A1**
(43) Date de publication de la demande: **03.08.2005**
(21) Numéro de dépôt: 05290022.2
(22) Date de dépôt: 05.01.2005
(51) Int. Cl.: A61K 7/13

(54) **Composition de teinture des fibres kératiniques contenant une alcool oxydase et un colorant direct à fonction acide ou son sel**

(30) Priorité: 28.01.2004 FR 0400784
(71) Demandeur: L'OREAL, 75008 Paris (FR)
(72) Inventeur: Plos, Grégory, Tokyo 158-0097 (JP)
(74) Mandataire: Dossmann, Gérard

(57) **Abrégé**

La présente demande concerne une composition pour la teinture des fibres kératiniques, en particulier des fibres kératiniques humaines telles que les cheveux, contenant, dans un milieu approprié pour la teinture, au moins un précurseur de colorant d'oxydation, au moins une enzyme alcool oxydase, au moins un substrat pour ladite enzyme et au moins un colorant direct à fonction acide. Cette demande concerne encore un procédé de teinture des fibres kératiniques qui consiste à appliquer cette composition ainsi qu'un "kit" de teinture.

## Description

La présente invention a pour objet une composition pour la teinture des fibres kératiniques, en particulier des fibres kératiniques humaines telles que les cheveux, contenant dans un milieu approprié pour la teinture, au moins un précurseur de colorant d'oxydation, au moins une enzyme alcool oxydase, au moins un substrat pour ladite enzyme, ainsi qu'un colorant direct à fonction acide ou son sel.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

La coloration est généralement réalisée en milieu fortement alcalin, en présence de peroxyde d'hydrogène. Toutefois, l'utilisation des milieux alcalins en présence de peroxyde d'hydrogène présente pour inconvénient d'entraîner une dégradation non négligeable des fibres, ainsi qu'une décoloration des fibres kératiniques qui n'est pas toujours souhaitable.

De plus, ce type de composition présente l'inconvénient de réaliser le mélange entre l'eau oxygénée et le support de coloration au moment de l'application de ladite composition sur les fibres kératiniques.

La coloration d'oxydation des fibres kératiniques peut également être réalisée à l'aide de systèmes oxydants différents du peroxyde d'hydrogène tels que des systèmes enzymatiques, en particulier avec des enzymes de type oxydase à 2 électrons. Ainsi la demande de brevet FR 2 769 219 décrit l'utilisation d'une enzyme uricase et de son substrat l'acide urique en coloration d'oxydation pour la teinture de fibres kératiniques. Ces enzymes catalysent l'oxydation d'un substrat par l'oxygénation de l'air pour générer un ou plusieurs produits d'oxydation, ainsi que de l'eau oxygénée. L'eau oxygénée générée peut être utilisée pour oxyder des précurseurs de colorants d'oxydation et par conséquent, produire de la couleur sur le cheveu. Ce système permet d'envisager une coloration d'oxydation sans mélange au moment de l'emploi. Cependant, les formulations de teintures utilisant l'alcool oxydase, bien qu'étant mises en oeuvre dans des conditions n'entraînant pas une dégradation du cheveu comparable à celle engendrée par les formulations utilisant de l'eau oxygénée et bien qu'offrant la possibilité d'être formulées en tout en un, conduisent à des colorations encore insuffisantes à la fois sur le plan de l'homogénéité de la couleur, de la puissance tinctoriale et de la chromaticité.

La demande EP-A-0 310 675 décrit l'utilisation de précurseur de colorant d'oxydation de type benzénique en association avec des enzymes telles que la pyranose-oxydase, la glucose-oxydase. Les compositions décrites dans ce brevet peuvent aussi comprendre des colorants directs. Toutefois, les colorations obtenues en utilisant ces compositions restent encore insuffisantes.

Le but de la présente invention est de fournir de nouvelles compositions pour la teinture des fibres kératiniques par coloration d'oxydation qui respectent la nature de la fibre kératinique, qui offrent la possibilité d'être formulées en tout en un et qui conduisent à des couleurs homogènes, puissantes et de forte chromaticité.

La demanderesse a découvert de nouvelles compositions contenant au moins un précurseur de colorant d'oxydation, au moins une enzyme de type alcool oxydase, au moins un substrat pour l'enzyme et au moins un colorant direct à fonction acide ou son sel, à l'exception des colorants directs quinoniques. Ces compositions selon la présente invention permettent l'obtention de teintures de couleurs chromatiques, puissantes, peu sélectives et résistantes, ces compositions sont capables d'engendrer des nuances variées de couleur intense et uniforme, sans dégradation signifiante du cheveu.

De préférence, la composition comprend au moins un précurseur de colorant d'oxydation.

D'autres caractéristiques, aspects, objets et avantages de la présente invention apparaîtront encore plus clairement à la lecture de la description et des exemples qui suivent.

Par "colorant direct à fonction acide ou son sel", on entend des colorants directs porteurs d'une fonction acide organique, telle que des fonctions acides carboxylique, sulfonique ou phosphorique, ou leurs sels avec des métaux alcalins ou alcalino-terreux, à l'exception des colorants directs quinoniques.

Les colorants directs à fonction acide ou leurs sels utilisables selon l'invention sont les colorants directs nitrés acides, les colorants azoïques acides, les colorants aziniques acides, les colorants triarylméthaniques acides, les colorants indoaminiques acides, les colorants naturels acides non quinoniques. De préférence, les colorants suivants seront utilisés :

| | |
|---|---|
| (C.1. 10316) | Sel de sodium de l'acide 2,4-dinitro-1-naphtol-7-sulfonique |
| (C.1. 10383) | Acid Orange 3 |
| (C.1. 13015) | Acid Yellow 9 / Food Yellow 2 |
| (C.1. 14780) | Direct Red 45 / Food Red 13 |
| (C.1. 13711) | Acid Black 52 |
| (C.1. 13065) | Acid Yellow 36 |
| (C.1. 14700) | Sel de sodium de l'acide 1-hydroxy-2-(2',4'-xylyl-5-sufonatoazo)-naphtalène-4-sulfonique / Food Red 1 |
| (C.1. 14720) | Acid Red 14/ Food Red 3/ Mordant Blue 79 |
| (C. 1. 14805) | Acid Brown 4 |
| (C.1. 15510) | Acid Orange 7 / Pigment Orange 17 / Solvent Orange 49 |
| (C.1. 15985) | Food Yellow 3 / Pigment Yellow 104 |
| (C.1. 16185) | Acid Red 27 / Food Red 9 |
| (C.1. 16230) | Acid Orange 10 / Food Orange 4 |
| (C.1. 16250) | Acid Red 44 |
| (C.1. 17200) | Acid Red 33 / Food Red 12 |
| (C.1. 13683) | Sel de sodium de l'acide 1-(3'-nitro-5'-sulfo-6'-oxophénylazo)-8-acétamido-naphtalène-3,6-disulfonique / Food Red 11 |
| (C.1. 18065) | Sel de sodium de l'acide 1-hydroxy-2-(2'-méthyl phénylazo)-8-acétamido-naphtalene-3,6-disulfonique / Acid Red 35 |
| (C.1. 19125) | Acid Violet 3 |
| (C.I. 18055) | Acid Red 35 |
| (C.I. 18055) | Acid Violet 7 |
| (C.1. 18130) | Acid Red 135 |
| (C.1. 19130) | Acid Yellow 27 |
| (C.1. 19140) | Acid Yellow 23 / Food Yellow 4 |
| (C.1. 20170) | 4'-(sulfonato-2",4"-diméthyl)-bis-(2,6-phénylazo)-1,3-dihydroxy benzène / Acid Orange 24 |
| (C.1. 20470) | Acid Black 1 (Sel de sodium de l'acide 1-amino-2-(4'-nitrophénylazo)-7-phénylazo-8-hydroxy-naphtalène-3,6-disulfonique) |
| (C.1. 23266) | (4-((4-méthylphényl) sulfonyloxy)-phénylazo)2,2'-diméthyl-4-((2-hydroxy-5,8-disulfonato)naphtylazo)biphényle / Acid Red 111 |
| (C.1. 27755) | Food Black 2 |
| (C.1. 25440) | 1-(4'-sulfonatophénylazo)-4-((2"-hydroxy-3"-acétylamino-6",8"-disulfonato)naphtylazo)-6-sulfonatonaphtalène (sel tétrasodique) / Food Black 1 |
| (C.1. 42080) | Acide 4-β-hydroxyéthylamino-3-nitrobenzènesulfonique |
| (C.I. 42090) | Acid Blue 9 |
| (C.1. 47005) | (5',6' ou 7')-sulfonato-6'-méthylquinoléine-2,2',Δ-1,3-indanedione / Acid Yellow 3 |
| (C.1. 14710) | Sel de sodium de l'acide 4-hydroxy-3((2-méthoxy phényl)-azo)-1-naphtalène sulfonique / Acid Red 4 |
| | Acide 2-pipéridino 5-nitro benzène sulfonique |
| | Acide 2(4'-N,N(2"-hydroxyéthyl)amino-2'-nitro)aniline éthane sulfonique |
| | Acide 4-β-hydroxyéthylamino-3-nitrobenzène sulfonique |
| | Acid Violet 49 |
| | Acid Blue 7 |
| | Acid Blue 156 |
| | Acid Blue 317 |

La plupart de ces colorants sont décrits en particulier dans le Color Index publié par The Society of Dyers ans Colorists, P.O. Box 244, Perkin House, 82 Grattan Road, Bradford, Yorkshire, BD1 2JBN England.

Le ou les colorants directs selon l'invention représentent de préférence de 0,001 à 20% en poids environ du poids total de la composition et encore plus préférentiellement, de 0,005 à 10% en poids environ.

Dans le cadre de la présente invention, les enzymes alcool oxydases pouvant être utilisées dans la composition tinctoriale conforme à l'invention appartiennent à la classe E.C.1.1.3 de la nomenclature des enzymes, (voir Enzyme Nomenclature, Academic Press Inc ; 1992).

Lesdites enzymes peuvent notamment être choisies parmi les alcool primaire oxydases ( EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7) encore appelées aryl alcool oxydases.

De préférence, l'enzyme utilisée dans la composition selon l'invention est une alcool primaire oxydase (EC 1.1.3.13).

Les enzymes alcool oxydases forment une classe particulière d'enzymes oxydo-réductase à 2 électrons.

L'enzyme alcool oxydase utilisée dans la composition tinctoriale selon l'invention peut être issue d'un extrait de végétaux, d'animaux, de microorganismes (bactérie, champignon, levure, microalgue ou virus), de cellules différenciées ou dédifférenciées, obtenues *in vivo* ou *in vitro,* modifiées ou non modifiées génétiquement, ou synthétiques (obtenues par synthèse chimique ou biotechnologique).

A titre d'exemples, on peut citer en particulier les enzymes extraites des espèces suivantes : *Pinus, Gastropode, Manduca, Pichia, Candida, Pleurotus, Pseudomonas, Rhodococcus, Aspergillus, Kamagataella, Phanerochaete, Polyporus, Hansenula, Poria, Penicillium*, et de façon encore plus particulière les espèces suivantes : *Pinus strobus*, qui est une espèce d'origine végétale, *Gastropode mollusc, Manduca sexta,* qui sont d'origine animale, *Pichia sp. (pastoris, methanolica, angusta)* et *Candida sp. (boidinii, albicans, tropicalis),* qui sont des levures, *Pleurotus pulmonarius Aspergillus niger, Kamagataella pastoris, Phanerochaete chrysosporium, Polyporus obtusus, Hansenula polymorpha, Poria contigua, Penicillium simplicissimum*, qui sont des champignons, et *Pseudomonas pseudoalcaligenes, Rhodococcus erythropolis*, qui sont des bactéries.

De préférence, l'alcool oxydase utilisée provient de la souche *Pichia pastoris*.

Généralement, la concentration en enzyme alcool oxydase utilisée dans la composition tinctorial est comprise entre 0,05 et 20% en poids par rapport au poids total de ladite composition, de préférence entre 0,1 et 10%, et plus particulièrement entre 0,5 et 8% en poids par rapport au poids total de la composition tinctoriale.

L'activité enzymatique des enzymes alcool oxydases utilisées conformément à l'invention peut être définie à partir de l'oxydation du donneur en condition aérobie. L'unité U correspond à la quantité d'enzyme conduisant à la génération de 1 µmole de peroxyde d'hydrogène par minute à un pH donné et à une température de 25°C.

De façon préférentielle, la quantité en enzyme alcool oxydase utilisée dans la composition tinctoriale est comprise entre 10³U et 10⁵U, et de préférence entre 2 10³ et 2 10⁴U pour 100g de composition tinctoriale.

Le ou les substrats pour ladite enzyme sont encore appelés donneurs pour l'enzyme. La nature de ce substrat varie en fonction de la nature de l'enzyme alcool oxydase qui est utilisée. Le substrat pour l'enzyme dans les compositions de l'invention sera de préférence un alcool choisi parmi les alcools primaires, secondaires, ramifiés ou non, saturés ou non, substitués ou non, les alcools à longue chaîne hydrocarbonée et les alcools aromatiques. Par exemple à titre de donneur pour les alcool primaire oxydases, on peut citer les alcools primaires contenant de 1 à 6 atomes de carbone ; à titre de donneur pour les aryl alcool oxydases : l'alcool benzylique, le 4-terbutyl benzylique alcool, le 3-hydroxy-4- méthoxybenzyl alcool, le vératryl alcool, le 4-méthoxybenzyl alcool, l'alcool cinnamique ; le 2,4 hexadiéne-1-ol peut également être utilisé en tant que donneur pour les aryl alcool oxydases.

Selon une autre variante de l'invention, le substrat pour l'enzyme est un composé portant au moins une fonction alcool aliphatique ou aromatique, approprié pour réagir avec l'enzyme utilisée. Le composé portant au moins une fonction alcool aliphatique ou aromatique peut notamment être un précurseur de colorant d'oxydation ou encore un adjuvant cosmétiquement acceptable par exemple un polymère, un tensioactif, un conservateur porteur d'au moins une fonction alcool. De façon encore plus préférée, le substrat pour l'enzyme est un précurseur de colorant d'oxydation portant au moins une fonction alcool aliphatique ou aromatique. Par exemple, la N-(β-hydroxypropyl)-paraphénylènediamine qui porte une fonction alcool primaire peut avoir la fonction de base d'oxydation et de substrat pour l'alcool oxydase. De même, les coupleurs d'oxydation, tels que le méta- ou le para-aminophénol, peuvent remplir les deux fonctions. De tels précurseurs sont décrits ci-après. Dans cette variante, l'utilisation d'autres substrats de l'enzyme est facultative.

Ainsi la présente demande vise une composition pour la teinture des fibres kératiniques, en particulier des fibres kératiniques humaines telles que les cheveux, contenant, dans un milieu approprié pour la teinture, au moins les composés suivants : un précurseur de colorant d'oxydation ; au moins une enzyme alcool oxydase ; au moins un substrat, portant une fonction alcool, pour ladite enzyme et au moins un colorant direct à fonction acide ou son sel, ledit substrat pouvant être substitué (c'est-à-dire remplacé) en tout ou en partie par le précurseur de colorant d'oxydation dans le cas où il porte au moins une fonction alcool aliphatique ou aromatique.

L'utilisation de la composition conforme à l'invention permet de diminuer les risques associés à la manipulation de peroxyde d'hydrogène. De plus, la concentration en conservateurs des compositions selon la présente invention peut être diminuée par l'apport de composés possédant une fonction alcool qui possèdent également des propriétés de conservateur.

Généralement, la concentration en alcools est comprise entre 0,01% et 60% en poids par rapport au poids total de la composition et de préférence comprise entre 0,05% et 30% en poids par rapport au poids total de la composition.

Les bases d'oxydation classiques peuvent notamment être choisies parmi les paraphénylènediamines, les bis-phénylalkylènediamines, les para-aminophénols, les ortho-aminophénols et les bases hétérocycliques et leurs sels d'addition.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6-dichlorophénol, le 4-amino-6[(5'-amino-2'-hydroxy-3'-méthyl)phénylméthyl]2-méthylphénol, le bis-(5'-amino-2'-hydroxyl)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide. D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxypyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diaminopyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2-hydroxy 4,5,6-triaminopyrimidine, la 2,4-dihydroxy 5,6-diaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Généralement la concentration de la ou des bases d'oxydation est comprise entre 0,0001 et 20%, de préférence entre 0,005 et 6% en poids par rapport au poids total de la composition.

Parmi les coupleurs d'oxydation classiques, on peut notamment citer les métaphénylènediamines, les méta-aminophénols, les métadiphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20% , de préférence entre 0,005 et 6% en poids par rapport au poids total de la composition.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Les sels d'addition utilisables dans le cadre de l'invention sont par exemple choisis parmi les sels d'addition avec la soude, la potasse, l'ammoniaque, les amines ou les alcanolamines.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des tensioactifs, des agents de conditionnement tels que par exemple des silicones volatiles ou non volatiles, modifiées ou non modifiées, des polymères cationiques, des cations, des agents filmogènes, des polymères épaississants, des céramides, des agents conservateurs, des agents opacifiants, des vitamines ou provitamines, des colorants directs autres que des colorants directs à fonction acide selon l'invention.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. Ce solvant peut être le cas échéant un substrat de l'enzyme comme l'éthanol et l'isopropanol. Cela peut être également un composé non substrat de l'enzyme tel que les éthers de polyols comme le 2-butoxyéthanol, le propylèneglycol, le monométhyléther de propylèneglycol, le monoéthyléther et le monométhyléther du diéthylèneglycol, le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 6 et 11 environ, et de préférence entre 7 et 10 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 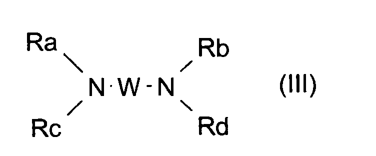 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale peut se présenter sous des formes diverses, telles que sous forme de liquides, de crèmes, de gels, ou sous toute autre forme appropriée pour réaliser une teinture des fibres kératiniques, et notamment des cheveux humains.

Lorsque les colorants d'oxydation et la ou les alcool oxydases sont présents au sein de la même composition prête à l'emploi, ladite composition est de préférence exempte d'oxygène gazeux de manière à éviter toute oxydation prématurée du ou des colorants d'oxydation.

L'invention a également pour objet un procédé de teinture des fibres kératiniques, et en particulier des fibres kératiniques humaines telles que les cheveux, tel qu'on applique sur ces fibres au moins une composition pour la teinture selon l'invention, pendant une durée suffisante pour développer la coloration désirée.

Au contact de l'oxygène de l'air, la couleur est révélée par la mise en présence de l'enzyme alcool oxydase et de son substrat.

La composition est appliquée sur les fibres kératiniques. Après un temps de pose de 3 à 60 minutes environ, de préférence 5 à 40 minutes environ, les fibres kératiniques sont rincées, lavées au shampooing, rincées à nouveau puis séchées.

Lorsque la composition pour la teinture est une composition sous une forme prête à l'emploi, elle comprend, dans un milieu approprié pour la teinture des fibres kératiniques, au moins un précurseur de colorant d'oxydation, au moins une enzyme alcool oxydase, au moins un substrat pour ladite enzyme et au moins un colorant direct acide, l'ensemble est alors stocké sous forme anaérobie, exempte d'oxygène gazeux.

Selon une variante, ce procédé comporte une étape préliminaire consistant à stocker sous forme séparée, d'une part une composition (A) comprenant, dans un milieu approprié pour la teinture des fibres kératiniques, au moins un précurseur de colorant d'oxydation, et d'autre part, une composition (B) renfermant, dans un milieu approprié pour la teinture des fibres kératiniques, au moins une enzyme alcool oxydase, la composition (A) et/ou la composition (B) contenant au moins un substrat pour ladite enzyme, et la composition (A) et/ou la composition (B) contenant au moins un colorant direct à fonction acide non quinonique puis à procéder au mélange des compositions (A) et (B) au moment de l'emploi avant d'appliquer ce mélange sur les fibres kératiniques.

De préférence, ce procédé comporte une étape préliminaire consistant à stocker sous forme séparée, d'une part une composition (A) comprenant, dans un milieu approprié pour la teinture des fibres kératiniques, au moins un précurseur de colorant d'oxydation, au moins un substrat pour ladite enzyme et au moins un colorant direct à fonction acide non quinonique et d'autre part, une composition (B) renfermant, dans un milieu approprié pour la teinture des fibres kératiniques, au moins une enzyme alcool oxydase, puis à procéder au mélange des compositions (A) et (B) au moment de l'emploi avant d'appliquer ce mélange sur les fibres kératiniques.

La couleur peut être révélée à pH acide, neutre ou alcalin. Dans le cas où le procédé est mis en oeuvre au moyen d'une composition (A) comprenant au moins un précurseur de colorant d'oxydation, au moins un substrat pour ladite enzyme et au moins un colorant direct à fonction acide non quinonique et d'une composition (B) renfermant au moins une enzyme alcool oxydase, l'enzyme peut être ajoutée à la composition de l'invention juste au moment de l'emploi ou peut être mis en oeuvre à partir d'une composition la contenant, appliquée simultanément ou séquentiellement à la composition de l'invention.

Cette composition (dite composition oxydante) peut également renfermer divers adjuvants utilisés classiquement dans les compositions pour la teinture des cheveux et tels que définis précédemment.

Le pH de la composition (B) dite oxydante est tel qu'après mélange avec la composition tinctoriale (A), le pH de la composition résultante appliquée sur les fibres kératiniques varie de préférence entre 6 et 11 environ, et encore plus préférentiellement entre 7 et 10. Il peut être ajusté à la valeur désirée au moyen d'agents acidifiants ou alcalinisants habituellement utilisés en teinture des fibres kératiniques et tels que définis précédemment.

De préférence, l'application de la composition selon l'invention est effectuée à une température comprise entre la température ambiante et 220°C, de préférence entre la température ambiante et 60°C.

Un autre objet de l'invention est un dispositif à plusieurs compartiments ou "kit" de teinture dans lequel un premier compartiment renferme la composition (A) telle que définie ci-dessus et un deuxième compartiment renferme la composition (B) telle que définie ci-dessus. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLE

La demanderesse a préparé la composition suivante conformément à l'invention.

| | |
|---|---|
| Acid Black 1 (colorant direct à fonction acide) | 0,4 g |
| Ethanol (substrat donneur) | 25g |
| Paraphénylènediamine (précuseur de coloration) | 3. 10⁻³ mole |
| Métaaminophénol (coupleur) | 3. 10⁻³ mole |
| Alcool oxydase | 20000unités |
| 2-amino-2-méthyl-1-propanol | Qs pH 7 |
| Eau distillé | qsp 100g |

L'alcool oxydase utilisée est celle commercialisée par la société Biozyme Laboratories sous forme liquide à la concentration de 1980 unités/ml.

L'unité U correspond à la quantité d'enzyme conduisant à la génération de 1 µmole de peroxyde d'hydrogène par minute à pH 7,5 (tampon phosphate 100mM) et à une température de 25°C.

On applique les compositions ci-dessus sur des mèches de cheveux gris naturels et permanentés à 90% de blancs et on laisse poser pendant 30 minutes. Le rapport de bain est fixé à 5. L'alcool oxydase est ajoutée extemporanément. Les cheveux ont ensuite été rincés, lavés avec un shampooing standard, puis séchés.

## Revendications

1. Composition pour la teinture des fibres kératiniques, en particulier des fibres kératiniques humaines telles que les cheveux, contenant, dans un milieu approprié pour la teinture, au moins les composés suivants : un précurseur de colorant d'oxydation ; au moins une enzyme alcool oxydase ; au moins un substrat, portant une fonction alcool, pour ladite enzyme et au moins un colorant direct à fonction acide ou un de ses sels, à l'exception des colorants directs quinoniques, ledit substrat pouvant être substitué en tout ou en partie par le précurseur de colorant d'oxydation dans le cas où ledit précurseur porte au moins une fonction alcool aliphatique ou aromatique.

2. Composition selon la revendication 1, **caractérisée en ce qu'**elle contient des colorants directs à fonction acide choisis parmi les colorants directs nitrés acides, les colorants azoïques acides, les colorants aziniques acides, les colorants triarylméthaniques acides, les colorants indoaminiques acides, les colorants naturels acides non quinoniques.

3. Composition selon la revendication 2, **caractérisée en ce que** le colorant direct à fonction acide ou son sel est choisi par les colorants suivants :
| | |
|---|---|
| (C.1. 10316) | Sel de sodium de l'acide 2,4-dinitro-1-naphtol-7-sulfonique |
| (C.1. 10383) | Acid Orange 3 |
| (C.1. 13015) | Acid Yellow 9 / Food Yellow 2 |
| (C.1. 14780) | Direct Red 45 / Food Red 13 |
| (C.1. 13711) | Acid Black 52 |
| (C.1. 13065) | Acid Yellow 36 |
| (C.1. 14700) | Sel de sodium de l'acide 1-hydroxy-2-(2',4'-xylyl-5-sufonatoazo)-naphtalène-4-sulfonique / Food Red 1 |
| (C.1. 14720) | Acid Red 14/ Food Red 3/ Mordant Blue 79 |
| (C. 1. 14805) | Acid Brown 4 |
| (C.1. 15510) | Acid Orange 7 / Pigment Orange 17 / Solvent Orange 49 |
| (C.1. 15985) | Food Yellow 3 / Pigment Yellow 104 |
| (C.1. 16185) | Acid Red 27 / Food Red 9 |
| (C.1. 16230) | Acid Orange 10 / Food Orange 4 |
| (C.1. 16250) | Acid Red 44 |
| (C.1. 17200) | Acid Red 33 / Food Red 12 |
| (C.1. 13683) | Sel de sodium de l'acide 1-(3'-nitro-5'-sulfo-6'-oxophénylazo)-8-acétamido-naphtalène-3,6-disulfonique / Food Red 11 |
| (C.1. 18065) | Sel de sodium de l'acide 1-hydroxy-2-(2'-méthyl phénylazo)-8-acétamido-naphtalene-3,6-disulfonique / Acid Red 35 |
| (C.1. 19125) | Acid Violet 3 |
| (C.I. 18055) | Acid Red 35 |
| (C.I. 18055) | Acid Violet 7 |
| (C.1. 18130) | Acid Red 135 |
| (C.1. 19130) | Acid Yellow 27 |
| (C.1. 19140) | Acid Yellow 23 / Food Yellow 4 |
| (C.1. 20170) | 4'-(sulfonato-2",4"-diméthyl)-bis-(2,6-phénylazo)-1,3-dihydroxy benzène / Acid Orange 24 |
| (C.1. 20470) | Acid Black 1 (Sel de sodium de l'acide 1-amino-2-(4'-nitrophénylazo)-7-phénylazo-8-hydroxy-naphtalène-3,6-disulfonique) |
| (C.1. 23266) | (4-((4-méthylphényl) sulfonyloxy)-phénylazo)2,2'-diméthyl-4-((2-hydroxy-5,8-disulfonato)naphtylazo)biphényle / Acid Red 111 |
| (C.1. 27755) | Food Black 2 |
| (C.1. 25440) | 1-(4'-sulfonatophénylazo)-4-((2"-hydroxy-3"-acétylamino-6",8"-disulfonato)naphtylazo)-6-sulfonatonaphtalène (sel tétrasodique) / Food Black 1 |
| (C.1. 42080) | Acide 4-β-hydroxyéthylamino-3-nitrobenzènesulfonique |
| (C.I. 42090) | Acid Blue 9 |
| (C.1. 47005) | (5',6' ou 7')-sulfonato-6'-méthylquinoléine-2,2',Δ-1,3-indanedione |
| (C.1. 14710) | Sel de sodium de l'acide 4-hydroxy-3((2-méthoxy phényl)-azo)-1-naphtalène sulfonique / acid red 4 |
| | Acide 2-pipéridino 5-nitro benzène sulfonique |
| | Acide 2(4'-N,N(2"-hydroxyéthyl)amino-2'-nitro)aniline éthane sulfonique |
| | Acide 4-β-hydroxyéthylamino-3-nitrobenzène sulfonique |
| | Acid Violet 49 |
| | Acid Blue 7 |
| | Acid Blue 156 |
| | Acid Blue 317 |

4. Composition selon l'une quelconque des revendications précédentes, **caractérisée en ce que** les colorants directs à fonction acide ou leurs sels représentent de préférence de 0,001 à 20% en poids environ du poids total de la composition et encore plus préférentiellement, de 0,005 à 10% en poids environ.

5. Composition selon l'une quelconque des revendications précédentes, **caractérisée en ce que** l'enzyme alcool oxydase appartient à la classe E.C.1.1.3.

6. Composition selon la revendication 5, **caractérisée en ce que** l'enzyme alcool oxydase est choisie parmi les alcool primaire oxydases (EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7).

7. Composition selon la revendication 6, **caractérisée en ce que** l'enzyme alcool oxydase est issue d'une des espèces suivantes : *Rhodococcus erythropolis, Pseudomonas pseudoalcaligenes, Aspergillus niger, Kamagataella pastoris, Phanerochaete chrysosporium, Polyporus obtusus, Hansenula polymorpha, Poria contigua, Penicillium simplicissimum, Pleurotus pulmonarius, Pichia sp. (pastoris, methanolica, angusta)* et *Candida sp. (boidinii, albicans, tropicalis)* , *Pinus strobus, Gastropode mollusc, Manduca sexta*.

8. Composition selon la revendication 7, **caractérisée en ce que** l'enzyme alcool oxydase est la *Pichia pastoris*.

9. Composition selon l'une quelconque des revendications précédentes, **caractérisée en ce que** la concentration en enzyme alcool oxydase est comprise entre 0,05 et 20%, de préférence entre 0,1 et 10%, et plus particulièrement entre 0,5 et 8% en poids par rapport au poids total de ladite composition.

10. Composition selon l'une quelconque des revendications précédentes, **caractérisée en ce que** la concentration en enzyme alcool oxydase est comprise entre 10³U et 10⁵U, de préférence entre 2.10³U et 5.10⁴U, pour 100g de la composition tinctoriale.

11. Composition selon l'une des revendications précédentes, **caractérisée en ce que** le substrat pour l'enzyme est un alcool choisi parmi les alcools primaires, secondaires, ramifiés ou non, saturés ou non, substitués ou non, les alcools à longue chaîne hydrocarbonée et les alcools aromatiques.

12. Composition selon la revendication 11, **caractérisée en ce que** la concentration du ou des substrats de l'enzyme est comprise entre 0,01% et 60% en poids par rapport au poids total de la composition et de préférence comprise entre 0,05% et 30% en poids par rapport au poids total de la composition.

13. Composition selon l'une des revendications précédentes, **caractérisée en ce qu'**elle comprend en tant que précurseur de colorant d'oxydation une base d'oxydation classique choisie parmi les paraphénylènediamines, les bis-phénylalkylènediamines, les para-aminophénols, les ortho-aminophénols, les bases hétérocycliques et leurs sels d'addition.

14. Composition selon la revendication 13, **caractérisée en ce que** la concentration de la ou des bases d'oxydation est comprise entre 0,0001 et 20% en poids par rapport au poids total de la composition.

15. Composition selon l'une des revendications précédentes, **caractérisée en ce que** la composition comprend en tant que précurseur de colorant d'oxydation un coupleur d'oxydation classique choisi parmi les métaphénylènediamines, les métaaminophénols, les métadiphénols, les coupleurs naphtaléniques, les coupleurs hétérocycliques ainsi que leurs sels d'addition.

16. Composition selon la revendication 15, **caractérisée en ce que** la concentration du ou des coupleurs est comprise entre 0,0001 et 20 % en poids par rapport au poids total de la composition.

17. Composition selon l'une des revendications précédentes, **caractérisée par le fait qu'**elle renferme un ou plusieurs colorants directs autres que des colorants directs à fonction acide ou leurs sels, tels que définis dans les revendications 1 à 4.

18. Procédé de teinture des fibres kératiniques et en particulier des fibres kératiniques humaines telles que les cheveux, **caractérisé par le fait qu'**on applique sur lesdites fibres au moins une composition selon l'une des revendications 1 à 17, pendant une durée suffisante pour développer la coloration désirée.

19. Procédé selon la revendication 18, **caractérisé par le fait que** la composition est une composition prête à l'emploi comprenant, une composition selon l'une des revendications 1 à 17, l'ensemble étant stocké sous forme anaérobie, exempte d'oxygène gazeux.

20. Procédé selon la revendication 19, **caractérisé par le fait qu'**il comporte une étape préliminaire consistant à stocker sous forme séparée, d'une part une composition (A) comprenant, dans un milieu approprié pour la teinture, au moins un précurseur de colorant d'oxydation et d'autre part, une composition (B) renfermant, dans un milieu approprié pour les fibres kératiniques, au moins une enzyme alcool oxydase, la composition (A) et/ou la composition (B) contenant au moins un substrat pour ladite enzyme et la composition (A) et/ou la composition (B) contenant au moins un colorant direct à fonction acide ou son sel, puis à procéder au mélange des compositions (A) et (B) au moment de l'emploi avant d'appliquer ce mélange sur les fibres kératiniques.

21. Procédé selon la revendication 20, **caractérisé par le fait qu'**il comporte une étape préliminaire consistant à stocker sous forme séparée, d'une part une composition (A) comprenant, dans un milieu approprié pour la teinture, au moins un précurseur de colorant d'oxydation, au moins un substrat pour l'enzyme alcool oxydase et au moins un colorant direct à fonction acide ou son sel et d'autre part, une composition (B) renfermant, dans un milieu approprié pour les fibres kératiniques, au moins une enzyme alcool oxydase, puis à procéder au mélange des compositions (A) et (B) au moment de l'emploi avant d'appliquer ce mélange sur les fibres kératiniques.

22. Dispositif à plusieurs compartiments ou « Kit » de teinture, **caractérisé par le fait qu'**il comporte un premier compartiment renfermant la composition (A) telle que définie à l'une des revendications 20 ou 21 et un second compartiment renfermant la composition (B) telle que définie à l'une des revendications 20 ou 21.
